# EUROPEAN PATENT APPLICATION

(11) **EP 4 375 656 A1**
(43) Date of publication of application: **29.05.2024**
(21) Application number: 22845455.9
(22) Date of filing: 22.07.2022
(51) Int. Cl.: G01N 27/26

(54) **MINIATURIZED BIOSENSOR AND SENSING STRUCTURE THEREOF**

(30) Priority: 22.07.2021 US 202163224736 P
(71) Applicant: Bionime Corporation, Taichung City 40242 (TW)
(72) Inventor: HUANG, Chun-Mu, Taichung, Taiwan 40242 (CN); CHEN, Chieh-Hsing, Taichung, Taiwan 40242 (CN); CHEN, Pi-Hsuan, Taichung, Taiwan 40242 (CN); CHEN, Chi-Hao, Taichung, Taiwan 40242 (CN); CHANG, Heng-Chia, Taichung, Taiwan 40242 (CN)
(74) Representative: Patentanwaltskanzlei WILHELM & BECK
(86) International application number: PCT/CN2022/107409
(87) International publication number: WO 2023/001292

(57) **Abstract**

The present invention provides a sensing structure of a micro biosensor (10) for performing a measurement of a physiological parameter of a target analyte (310) of a biofluid and reducing an interference of an interferant (320) of the biofluid on the measurement by an electrochemical reaction. The sensing structure includes: a substrate (110) having a surface (111); a first working electrode (120) configured on the surface (111), and including an active surface; at least one second working electrode (130) configured on the surface (111) and adjacent to the first working electrode (120), for consuming the interferant (320) by the electrochemical reaction; and an isolated layer (150) configured with respect to the active surface to program a diffusive distribution of the interferant (320) when the biofluid flows through the second working electrode (130), wherein at least the interferant (320) of the biofluid passes through the second working electrode (130) over a time period and is consumed by the second working electrode (130) by the electrochemical reaction.

## Description

### FIELD OF THE INVENTION

The present invention is related to a micro biosensor and sensing structure thereof. Particularly, the present invention is related to a micro biosensor and sensing structure thereof which can limit a diffusive distribution of a biofluid.

### BACKGROUND OF THE INVENTION

The basic configuration of the continuous glucose monitoring system includes a biosensor and a transmitter. The biosensor measures a physiological signal in response to a glucose concentration in the body, and the measurement thereof is mostly based on an electrochemical process. Specifically, glucose is subjected to a catalysis reaction with glucose oxidase (GOx) to produce gluconolactone and a reduced glucose oxidase, followed by an electron transfer reaction between the reduced glucose oxidase and oxygen in a biological fluid of the body to produce hydrogen peroxide (H₂O₂) as a byproduct. The glucose concentration is then derived from an oxidation reaction of the byproduct H₂O₂.

However, if interferants, such as ascorbic acid (a major component of vitamin C), acetaminophen (a common analgesic ingredient), uric acid, protein, glucose analogs, or the like, which oxidation potentials are proximate to the oxidation potential of H₂O₂, are present in the blood or the tissue fluid, the measurement of glucose concentration will be adversely affected. Therefore, it is difficult to ensure that the physiological parameters of a subject are truly reflected by the measurement values and to maintain a long-term stability of the measured signal when the continuous glucose monitoring system is in operation.

At present, the aforesaid shortcomings are solved, for example, by providing a polymer membrane to filter out the interferants. However, it remains difficult to filter out the interferants completely. Alternatively, a plurality of working electrodes optionally coated with an enzyme or different types of enzymes are respectively applied with potentials to read a plurality of signals from the working electrodes. The signals are then processed, such as subtraction, to accurately obtain the physiological parameter of the target analyte. However, such conventional processes involving the manufacturing and use of the working electrodes are very complicated. In addition, it is not easy to find out an appropriate ratio for the subtraction.

### SUMMARY OF THE INVENTION

A micro biosensor of the present invention can be implanted under a skin to measure a physiological parameter of a target analyte of a biofluid, and includes a first working electrode for measuring the physiological parameter, a second working electrode for consuming interferants and an isolated layer. The isolated layer is configured to at least shield a part of the first working electrode to prevent the interferants in the biofluid from diffusing to the first working electrode directly, to ensure the biofluid passes through the second working electrode before reaching the first working electrode, so that the second working electrode consumes the interferants that affect the measurement in the biological fluid using an electrochemical reaction, and the first working electrode can obtain more accurate measurement results during measurement.

In accordance with another aspect of the present disclosure, a micro biosensor for implantation under a skin to measure a physiological parameter of a target analyte of a biofluid and reduce an interference of an interferant of the biofluid on the measurement by an electrochemical reaction is disclosed. The micro biosensor includes: a substrate being a sheet and having a first surface and a second surface which are oppositely configured; a first working electrode at least including a first sensing section configured on the first surface of the substrate, wherein the first sensing section of the first working electrode includes a first conductive material; at least one second working electrode configured on the first surface of the substrate, and including a second sensing section, wherein the second sensing section is configured adjacent to at least one side of the first sensing section, and the second sensing section of the second working electrode includes a second conductive material different from the first conductive material; a first functional membrane covering the first sensing section of the first working electrode and the second sensing section of the second working electrode, wherein the first functional membrane includes a chemical reagent at least covering a part of the first conductive material to define an active surface of the first sensing section, for reacting with the target analyte of the biofluid so as to obtain a resultant; and an isolated layer at least configured with respect to at least a part of the active surface of the first sensing section of the first working electrode to optimize a diffusive path of the interferant of the biofluid as one passing through the second sensing section of the second working electrode, wherein: the biofluid diffuses to the second sensing section over a time period, and then diffuses to the first sensing section after passing through the second sensing section; when the first working electrode is driven by a first working voltage, the first sensing section reacts with the resultant for outputting a physiological signal corresponding to the physiological parameter of the target analyte; and when the second working electrode is driven by a second working voltage, the second sensing section consumes the interferant of the biofluid by the electrochemical reaction during the time period, and a remaining part of the biofluid diffuses to the first sensing section of the first working electrode after passing through the second sensing section, for reducing the interference of the interferant to the physiological signal.

In accordance with one more aspect of the present disclosure, a micro biosensor for implantation under a skin to measure a physiological parameter of a target analyte of a biofluid and reduce an interference of an interferant of the biofluid on the measurement by an electrochemical reaction is disclosed. The micro biosensor includes: a substrate having a first surface and a second surface which are oppositely configured; a first working electrode at least including a first sensing section configured on the first surface of the substrate, for measuring the physiological parameter of the target analyte; at least one second working electrode configured on the first surface of the substrate, and including a second sensing section, wherein the second sensing section is configured adjacent to at least one side of the first sensing section for consuming the interferant by the electrochemical reaction; a first functional membrane covering the first sensing section of the first working electrode and the second sensing section of the second working electrode, wherein the first functional membrane includes a chemical reagent at least covering a part of the first sensing section to define an active surface, for reacting with the target analyte of the biofluid so as to obtain a resultant; and an isolated layer at least configured with respect to at least a part of the active surface to delineate a diffusive path of the interferant as one causing the biofluid to gain an increased opportunity to interact with the second sensing section of the second working electrode, wherein: when the first working electrode is driven by a first working voltage, the first sensing section reacts with the resultant for outputting a physiological signal corresponding to the physiological parameter of the target analyte; and when the second working electrode is driven by a second working voltage, the second sensing section consumes the interferant of the biofluid by the electrochemical reaction, and a remaining part of the biofluid diffuses to the first sensing section after passing through the second sensing section, for reducing the interference of the interferant to the physiological signal.

In accordance with one more aspect of the present disclosure, a sensing structure of a micro biosensor for implantation under a skin to measure a physiological parameter of a target analyte of a biofluid and reduce an interference of an interferant of the biofluid on the measurement by an electrochemical reaction is provided. The sensing structure includes: a substrate having a surface; a first working electrode configured on the surface of the substrate, and having an active surface; at least one second working electrode configured on the surface of the substrate and adjacent to at least one side of the first working electrode, for consuming the interferant by the electrochemical reaction; and an isolated layer at least configured with respect to at least a part of the active surface to program a diffusive distribution of the interferant when the biofluid flows through the second working electrode, wherein at least the interferant of the biofluid passes through the second working electrode over a time period and is consumed by the second working electrode by the electrochemical reaction.

### BRIEF DESCRIPTION OF THE DRAWINGS

Other objectives, advantages and efficacies of the present invention will be described in detail below taken from the preferred embodiments with reference to the accompanying drawings.
[Fig. 1A] shows a front schematic diagram of the first embodiment of the micro biosensor of the present invention.
[Fig. 1B] shows a back schematic diagram of the first embodiment of the micro biosensor of the present invention.
[Fig. 2A] shows a sectional schematic diagram of a cut view of the micro biosensor along the section line I-I in Figs. 1A and 1B.
[Fig. 2B] shows another sectional schematic diagram of a cut view of the micro biosensor along the section line I-I in Figs. 1A and 1B.
[Fig. 3] shows a schematic diagram of the measurement range of the first sensing section and the interference eliminating range of the second sensing section after the micro biosensor is driven.
[Fig. 4] shows a schematic diagram of the isolated layer in the micro biosensor of the present invention controlling the diffusive path of the interferant.
[Fig. 5A] shows a front schematic diagram of the second embodiment of the micro biosensor of the present invention.
[Fig. 5B] shows a back schematic diagram of the second embodiment of the micro biosensor of the present invention.
[Fig. 6] shows a sectional schematic diagram of a cut view of the micro biosensor along the section line II-II in Figs. 5A and 5B.
[Fig. 7] shows a schematic diagram of the configuration of another embodiment of the isolated layer of the micro biosensor of the present invention.
[Fig. 8] shows a schematic configuration diagram of another embodiment of the isolated layer of the micro biosensor of the present invention.
[Fig. 9] shows a front schematic diagram of the third embodiment of the micro biosensor of the present invention.
[Fig. 10] shows a front schematic diagram of the fourth embodiment of the sensing area of the sensing structure in the micro biosensor of the present invention.
[Fig. 11] shows a sectional schematic diagram of a cut view of the micro biosensor along the section line IV-IV in Fig. 10.
[Fig. 12] shows a schematic diagram of the configuration of the second functional membrane of the micro biosensor of the present invention.
[Fig. 13] shows a schematic diagram of the configuration of the filler of the micro biosensor of the present invention.

### DETAILED DESCRIPTION OF THE PREFERRED EMBODIMENTS

The present invention will now be described more specifically with reference to the following embodiments. It is to be noted that the following descriptions of preferred embodiments of this invention are presented herein for purpose of illustration and description only; they are not intended to be exhaustive or to be limited to the precise form disclosed. In the preferred embodiments, the same reference numeral represents the same element in each embodiment.

The micro biosensor of the present invention can be a sensor of a continuous glucose monitoring system, which is implanted under a skin of a living body to continuously measure physiological parameters of a target analyte in a biofluid. In addition, the term "target analyte" mentioned herein generally refers to any substance to be tested that exists in the living body, such as but not limited to glucose, lactose, uric acid, etc. The term "biofluid" may be but not limited to blood or interstitial fluid (ISF), and the term "physiological parameter" may be but not limited to concentration.

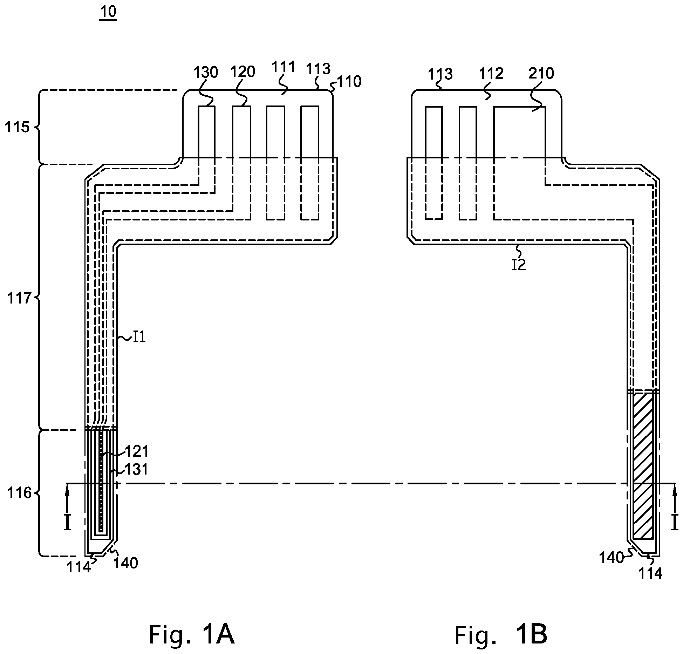

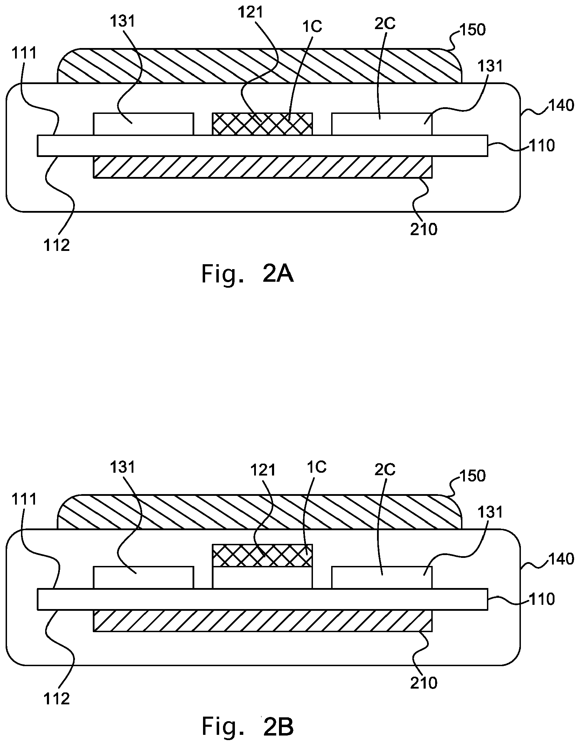

Please refer to Figs. 1A, 1B and 2A, wherein Figs. 1A and 2A respectively show front and back schematic diagrams of the first embodiment of the micro biosensor of the present invention, and Fig. 2A shows a sectional schematic diagram of a cut view of the micro biosensor along the section line I-I in Figs. 1A and 1B. In the first embodiment, a micro biosensor 10 of the present invention includes a substrate 110, a first working electrode 120, a second working electrode 130, a counter electrode 210, a first functional membrane 140 and an isolated layer 150.

The substrate 110 is a sheet, and has a first surface 111, a second surface 112 oppositely configured to the first surface 111, a first end 113 and a second end 114. Preferably, both the first surface 111 and the second surface 112 are flat planes for disposing the following electrodes. A signal output area 115, a sensing area 116 and an insulating area 117 are further defined on the substrate 110. The signal output area 115 is located at an area close to the first end 113, the sensing area 116 is located at an area close to the second end 114, and the insulating area 117 is covered by a first insulating layer I1 and located at an area between the signal output area 115 and the sensing area 116. The substrate 110 can be made of any material that can be used to manufacture electrode substrates and has flexibility and insulation, such as but not limited to, polyester, polyimide and other polymer materials. The polymer materials may be used alone or used as a mixture. The sensing structure of the present invention includes at least the first working electrode 120, the second working electrode 130, the first functional membrane 140 and the isolated layer 150 formed in the sensing area 116 and on the substrate 110.

The first working electrode 120 and the second working electrode 130 are configured on the first surface 111 of the substrate 110, and extended from the first end 113 to the second end 114, wherein a portion of the first working electrode 120 in the sensing area 116 is a first sensing section 121, and a portion of the second working electrode 120 in the sensing area 116 is a second sensing section 131. The first sensing section 121 at least has a first conductive material 1C, and the second sensing section 131 at least has a second conductive material 2C. The first conductive material 1C can be one of carbon, platinum, aluminum, gallium, gold, indium, iridium, iron, lead, magnesium, nickel, molybdenum, osmium, palladium, rhodium, silver, tin, titanium, zinc, silicon and zirconium, a derivative thereof (such as alloy, oxide or metal compound), or a combination thereof, and the second conductive material 2C can be the element or the derivative thereof exemplified for the first conductive material 1C.

It must be noted that, in the first embodiment, the first conductive material 1C is different from the second conductive material 2C. In order to obtain these structures, in the manufacturing process, the first conductive material 1C and the second conductive material 2C can be respectively formed on the first surface 111 of the substrate 110 at first and patterned into a pattern as shown in Fig. 1A, followed by the formation of the insulating layer I1 on the substrate 110 to define the signal output area 115, the sensing area 116 and the insulating area 117. In another embodiment, the second conductive material 2C can be defined as a pattern as shown in Fig. 1A without the pattern of the first sensing section 121 in the patterning step, and specifically, the first working electrode 120 further includes the second conductive material 2C which is only formed in the signal output area 115 and the insulating area 117 or extended at most into a part of the sensing area 116 in this embodiment. Then, the first conductive material 1C is directly formed on the area of the original first surface 111 where the first sensing section 121 is expected to be formed, and electrically connected to the other part of the first working electrode 120 (i.e., the second conductive material 2C) to finish the configuration of the first sensing section 121. The sectional schematic diagram of the sensing area 116 of the micro biosensor 10 in this embodiment is also shown as Fig. 2A.

However, the structures of the first working electrode 120 and the second working electrode 130 of the present invention are not limited to those shown in Fig. 2A. Please refer to Fig. 2B, which shows another sectional schematic diagram of a cut view of the micro biosensor along the section line I-I in Figs. 1A and 1B. In fig. 2B, the second conductive material 2C is first formed on the first surface 111 of the substrate 110, and then patterned into a pattern as shown in Fig. 1A. Specifically, the second conductive material 2C is divided into two separated areas, wherein one of the two areas extended from the first end 113 of the substrate 110 to the second end 114 and bent at the second end 114 to form the U-shape structure is preset as the second working electrode 130, and the other area extended from the first end 113 of the substrate 110 to the second end 114 and thus surrounded by the U-shaped structure is preset as the first working electrode 120. After the insulating layer 140 is covered on the substrate 110 and exposes the signal output area 115 and the sensing area 116, the first conductive material 1C is formed on the second conductive material 2C of the first working electrode 120 at the sensing area 116 to finish the manufacture of the first sensing section 121 of the first working electrode 120. However, although the figure does not show, the first conductive material 1C also can be only formed on the partial second conductive material 2C of the first working electrode 120 at the sensing area 116.

The second sensing section 131 of the present invention is adjacent to at least one side of the first sensing section 121, and a side of the second sensing section 131 extends along the at least one side of the first sensing section 121. In this embodiment, the second sensing section 131 extends along three sides of the first sensing section 121 to form a U-shape sensing section. In addition, the first sensing section 121 and the second sensing section 131 of the present invention maintain a positional relationship therebetween only via the first surface 111. Because the first sensing section 121 and the second sensing section 131 of the present invention are directly adjacent to each other, there are no intermediates, such as electrodes or connecting wires therebetween.

The first functional membrane 140 at least covers the first sensing section 121 of the first working electrode 120 and the second sensing section 131 of the second working electrode 130. Specifically, the first functional membrane 140 of the first embodiment surrounds the substrate 110, the first working electrode 120, the second working electrode 130 and the counter electrode 210. The first functional membrane 140 includes a chemical reagent at least covers a part of the first conductive material 1C of the first sensing section 121, so that a surface of the first sensing section 121 covered by the chemical reagent is defined as an active surface. In another embodiment, the chemical reagent can also cover the second conductive material 2C of the second sensing section 131 of the second working electrode 130, or even cover the second surface 112 of the substrate 110. That is to say, the chemical reagent can surround the sensing area 116. Basically, the chemical reagent includes at least one enzyme that reacts with the target analyte or catalyzes a reaction of the target analyte, such as, but not limited to, glucose oxidase, glucose dehydrogenase, etc. In addition to the chemical reagent, different functions, for example adjusting a detection sensitivity or anti-biofouling, of the first functional membrane 140 can be set upon the needs.

The first working electrode 120 of the micro biosensor 10 of the present invention is driven for measuring the physiological parameter of the target analyte of the biofluid. When the first working electrode 120 of the micro biosensor 10 is driven by the first working voltage, the first sensing section 121 has a first sensitivity to the resultant, so that the first conductive material 1C reacts with the resultant to generate a current signal. When the value of the current signal has a proportional relationship with the concentration of the resultant, the physiological signal corresponding to the physiological parameter is obtained. Therefore, in a range of the active surface of the first sensing section 121, the chemical reagent reacts with the target analyte of the biofluid so as to obtain the resultant, followed by the resultant reacting with the first conductive material 1C of the first sensing section 121 to produce the current signal, which corresponds to the physiological signal of the physiological parameter of the target analyte in the biofluid, and the physiological signal is transmitted to the signal output area 115 to be output.

Because the biofluid includes the target analyte and the interferant, the first conductive material 1C not only reacts with the resultant to produce the above-mentioned current signal, but also reacts with the interferant in the biofluid to produce an interfering current signal. The interfering current signal will be mixed with the current signal and output to interfere in the user's determination on the physiological signal. Similarly, when the second working electrode 130 is driven by the second working voltage, the second conductive material 2C of the second sensing section 131 has a second sensitivity to the resultant, so that the second conductive material 2C has an opportunity to react with the resultant to generate another current signal. That is to say, the second conductive material 2C consumes the resultant that should be measured by the first sensing section 121 of the first working electrode 120 to obtain the physiological parameter of the target analyte, so that the actual measured physiological parameter is affected. Therefore, in an embodiment, when the target analyte is glucose, the resultant is hydrogen peroxide and the physiological parameter is the glucose concentration, the first conductive material 1C should preferably be a material having the first sensitivity to hydrogen peroxide after being driven by the first working voltage. More preferably, the first conductive material 1C is selected from the group consisting of gold, platinum, palladium, iridium, and a combination thereof. The second conductive material 2C should preferably be a material having the second sensitivity to hydrogen peroxide that is less than the first sensitivity after being driven by the second working voltage. In particular, the second conductive material 2C is a material that almost has no sensitivity to hydrogen peroxide after being driven by the second working voltage, that is, the second sensitivity is close to 0 or equal to 0. In addition, the second sensing section 131 of the second working electrode 130 of the present invention provides an active surface for consuming the interferant. Therefore, the second conductive material 2C needs to be a material that almost has no sensitivity to hydrogen peroxide, but performs an oxidation reaction with the interferant to directly and continuously consume the interferant. In particular, the second conductive material 2C has a sensitivity to the interferant similar to the first conductive material 1C.

More specifically, in an embodiment of the present invention, the first conductive material 1C is platinum, the first working voltage ranges from 0.2 volts (V) to 0.8 volts (V) and preferably ranges from 0.4 V to 0.7 V, and the second conductive material 2C is carbon, the second working voltage ranges from 0.2 V to 0.8 V and preferably ranges from 0.4 V to 0.7 V In another embodiment of the present invention, the first conductive material 1C is platinum, and the second conductive material 2C is gold. It must be noted that the form of the aforementioned platinum can be platinum metal, platinum black, platinum paste, other platinum-containing materials, or a combination thereof. In addition, the value of the first working voltage can be the same as that of the second working voltage, but the invention is not limited thereto.

It must be noted that the term "drive" in the present invention means applying a voltage causing a potential of one electrode to be higher than that of the other electrode, so that the electrode with the higher potential starts the oxidation reaction. Therefore, the potential difference between the first working electrode 120 and the counter electrode 210 causing the first working electrode 120 to be driven is the first working voltage, and the potential difference between the second working electrode 130 and the counter electrode 210 causing the second electrode 130 to be driven is the second working voltage.

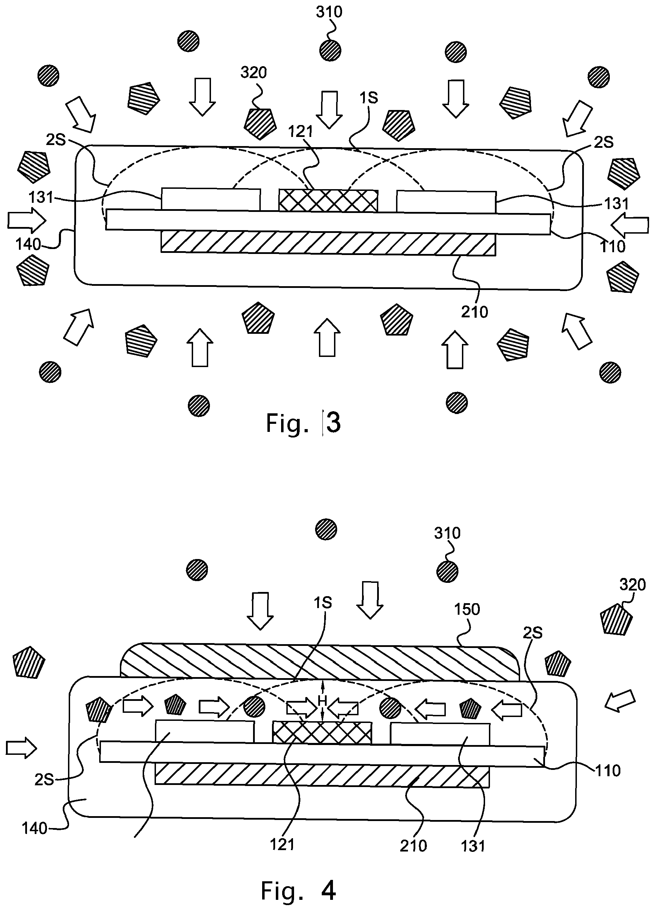

Please refer to Fig. 3, which is a schematic diagram of the measurement range of the first sensing section and the interference eliminating range of the second sensing section after the micro biosensor is driven. When the first working electrode 120 of the micro biosensor 10 is driven by the first working voltage, the first sensing section 121 produces a measurement range 1S, and when the second working electrode 130 of the micro biosensor 10 is driven by the second working voltage, each of the second sensing section 131 around the first sensing section 121 produces an interference eliminating range 2S. Because the second sensing section 131 is disposed adjacent to the sides of the first sensing section 121 and very close to the first sensing section 121, the interference eliminating ranges 2S touch the periphery of the first sensing section 121 and can at least partially overlap the measurement range 1S of the first sensing section 121, so that the second conductive material 2C can consume the interferant directly and continuously in the measurement range 1S of the first sensing section 121 by undergoing the electrochemical reaction with the interferant, so as to reduce the generation of the interfering current signal, and thereby reducing the influence of the interferant on the measurement of the first sensing section 121.

However, in Fig. 3, because the target analyte 310 and the interferant 320 will enter the measurement range 1S of the micro biosensor 10 in all directions, although the second sensing section 131 configured around the first sensing section 121 can consume the interferant 320, the interferant still has opportunities to approach the active surface of the first sensing section 121 from a non-overlapping range and then sensed.

Therefore, the micro biosensor 10 of the present invention includes the isolated layer 150, which is preferably configured on the first functional membrane 140. The position of the isolated layer 150 corresponds to at least a part of the first sensing section 121 of the first working electrode 120 to at least shield a part of the active surface, or even extends to at least shield a part of the second sensing section 131. Specifically, a configuration area of the isolated layer 150 may be 0.5-10 times the area of the active surface of the first sensing section 121, preferably 1-8 times, more preferably 2-6 times, and the most preferably 4-5 times.

In the first embodiment, the isolated layer 150 is configured to shield the top of the first sensing section 121 and the second sensing section 131, as shown in Fig. 2A. The isolated layer 150 can at least prevent the interferant 320, or even prevent both the target analyte 310 and the interferant 320, from directly diffusing to the active surface of the first sensing section 121 of the first working electrode 120. Taking the interferant as an example, the isolated layer 150 can isolate the interferant 320 to change the diffusive path or the diffusive distribution of the interferant 320. Specifically, the isolated layer 150 is configured to control the diffusive path of the interferant 320 of the biofluid to pass through the second sensing section 131 of the second working electrode 130 first, and a remaining part and the interferant that may not have been consumed in the biofluid then diffuse to the first sensing section 121 of the first working electrode 120 after passing through the second sensing section 131. Therefore, the diffusive path of the interferant 320 is optimized to pass through the interference eliminating range 2S of the second sensing section 131 located on the left and right sides of the first sensing sections 121 first, as shown in Fig. 4. In addition, the biofluid needs a time period to pass through the second sensing section 131 of the second working electrode 130 before diffusing to the first sensing section 121. The term "time period" refers to the time required for at least the interferant of the biofluid to diffuse to the second sensing section 131 of the second working electrode 130 along the diffusive path and pass through the second sensing section 131, or even further diffuse to the first sensing section 121. It must be understood that if the time period is too short, the second sensing section 131 has not enough time to consume the interferant, thereby affecting the interference eliminating effect, but if the time period is too long, the subsequent schedule of measuring the physiological signal by the first sensing section 121 will be delayed. Furthermore, a length of the time period can be determined by the length of the diffusive path, a diffusive range or a cross-sectional area of the diffusive path, or the properties of the material which the biofluid passes through. Specifically, the time period is ranged from 10 seconds to 15 minutes, preferably 3 minutes to 12 minutes, and more preferably 5 minutes to 8 minutes.

As for the target analyte, whether the target analyte can be allowed to pass through the isolated layer 150 is determined depending on the material and/or the thickness of the isolated layer 150. Therefore, the diffusive path of the target analyte 310 can be (1) passing through the second sensing section 131 of the second working electrode 130 first, and then diffusing to the first sensing section 121 of the first working electrode 120, and/or (2) directly diffusing to the active surface of the first sensing section 121 of the first working electrode 120 from the isolated layer 150 depending on the condition of the isolated layer 150. Due to the different characteristics of the conductive materials, the interferant 320 will be directly consumed by the second sensing section 131, and since most of the target analyte 310 can pass through the second sensing section 131 or directly reach the first sensing section 121, the target analyte 310 can be sensed in the sensing range 1S of the first sensing section 121. Therefore, the isolated layer 150 of the micro biosensor 10 of the present invention can ensure the interferant 320 is consumed by the second sensing section 131 before reaching the first sensing section 121, thereby effectively reducing the interference of the interferant 320 on the physiological signal measured by the first sensing section 131 to be less than or equal to an error range, such as 20%, preferably 10%, to increase the accuracy of the biological signal. Specifically, more than 90% interferants can be effectively consumed via the sensing structure provided by the present invention.

In addition, as mentioned in the preceding paragraph, the permeability of the isolated layer, such as the isolating object and the isolating degree, can be adjusted according to the choice of the material (such as the hydrophilicity or hydrophobicity of the material), the design of the thickness, or a combination thereof, to allow the substances such as glucose and oxygen to pass through the isolated layer and at least isolate the interferant, or completely isolate glucose, oxygen and the interferant from directly diffusing to the active surface of the first sensing section 121. For example, when the isolated layer with a high permeability is used, the thickness of the isolated layer with the high permeability should be larger than that of the isolated layer with a low permeability. In an embodiment, the isolated layer 150 can include poly-p-xylylene to stop the passage of glucose and the interferant. In another embodiment, the isolated layer 150 can include thermoplastic polyurethane, such as polycarbonate-based urethane, polyether based thermoplastic polyurethane, or a combination thereof, to at least stop the passage of the interferant. In another embodiment, the isolated layer 150 also can include a cellulosic derivative or a mixture of cellulosic derivatives, polyvinyl chloride, Nafion, or a combination thereof. Specifically, the thickness of the isolated layer 150 can range from 1 µm to 80 µm depending on the material used, preferably 3 µm to 24 µm, and more preferably 5 µm to 10 µm. In the previous embodiment, when the isolated layer 150 is composed of poly-p-xylylene, the thickness thereof is 1 µm; and when the isolated layer 150 is composed of polycarbonate-based urethane or its derives, for example polycarbonate based silicone elastomer, the thickness thereof ranges from 3 µm to 10 µm. In addition, the isolated layer 150 can be, but not limited to, manufactured on the first functional membrane 140 by a spraying process or a mask and screen printing. It should be additionally stated that the isolated layer of the present invention is not an insulation between the two electrodes on the substrate in the prior art or any insulation in the manufacturing process.

The first functional membrane 140 has a thickness H defined by a distance between the active surface of the first sensing section 121 and the active surface of the second sensing section 131 and the isolated layer 150, to have enough space to undergo the electrochemical reactions for the first sensing section 121 and the target analysis, and the second sensing section 131 and the interferant. The thickness H affects the sensitivity of the sensing. The thickness H is no less than 0.05 µm and no larger than 50 µm, preferably between 0.1 µm and 20 µm, more preferably between 2 µm and 8 µm, and the most preferably between 3 µm and 5 µm, to ensure and enhance the interference eliminating effect.

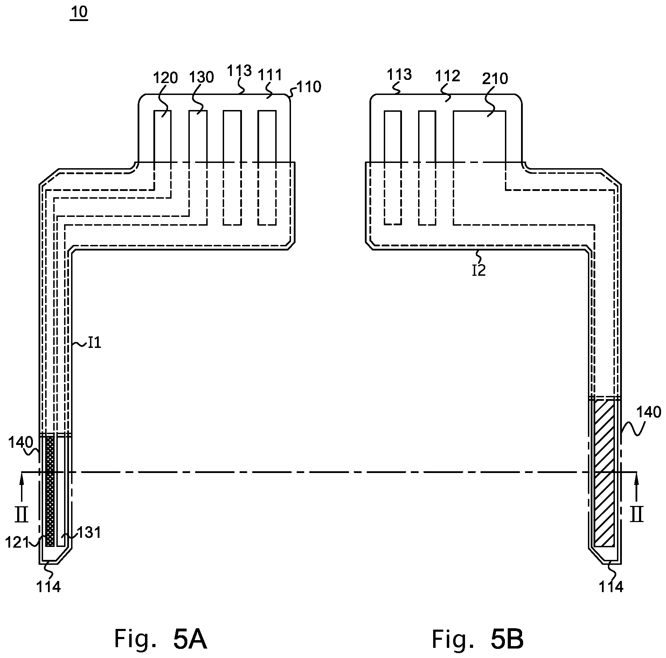

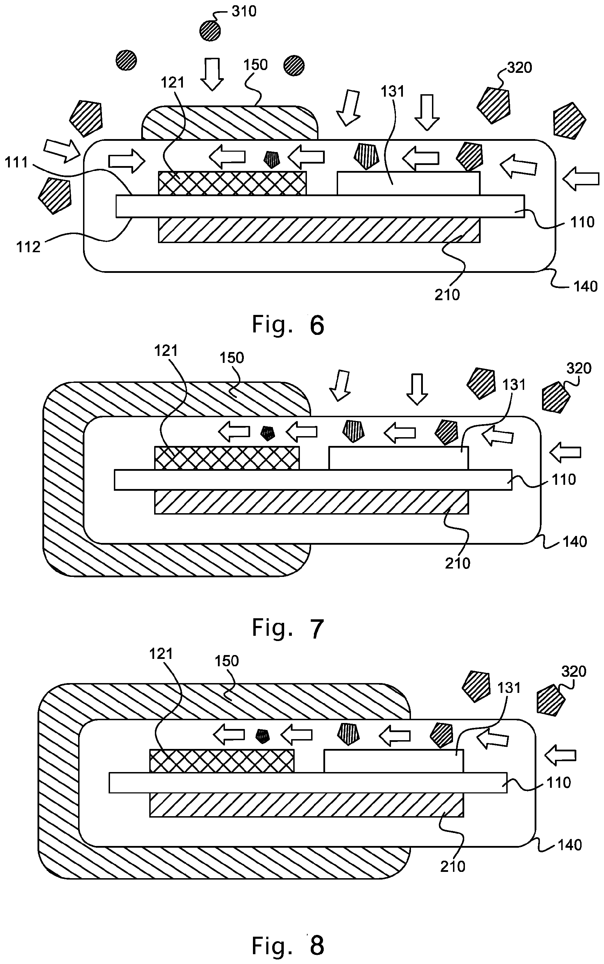

Please refer to Figs. 5A, 5B and 6, wherein Figs. 5A and 5A respectively show front and back schematic diagrams of the second embodiment of the micro biosensor of the present invention, and Fig. 6 shows a sectional schematic diagram of a cut view of the micro biosensor along the section line II-II in Figs. 5A and 5B. In the second embodiment, the first working electrode 120 and the second working electrode 130 extend from the first end 113 to the second end 114 of the substrate 110, and in the sensing area, the second sensing section 131 extends along one side of the first sensing section 121 without bending, so that the second sensing section 131 is only adjacent to the one side of the first sensing section 121. The isolated layer 150 is configured above the first functional membrane 140 and shields the top of the first working electrode 120 to limit and guide at least a part of the biological fluid to pass through the second sensing section 131 before reaching the first sensing section 121, so that a part of the interferant 320 is consumed. Therefore, the sensing of the target analyte 310 of the micro biosensor with the isolated layer 150 is more accurate than that of the micro biosensor without the isolated layer 150.

In another embodiment, the isolated layer 150 can be disposed to wrap the left side of the sensing structure as shown in Fig. 7, that is, the isolated layer 150 is extended from the position corresponding to the first sensing section 121, along the first functional membrane 140 to the counter electrode 210, to isolate the interferant 320 from directly entering the measurement range from the left side of the first sensing section 121 to affect the sensing result of the physiological parameter. In another embodiment, the isolated layer 150 can also be disposed to wrap about 2/3 of the sensing structure as shown in Fig. 8. That is, the isolated layer 150 is extended from the position corresponding to the first sensing section 121 and a part of the second sensing section 131, along the first functional membrane 140 to the counter electrode 210. Specifically, the isolated layer 150 can not only completely isolate the interferant 320 from directly entering the measurement range from the left side of the first sensing section 121 and the gap between the first sensing section 121 and the second sensing section 131, but also limit the interferant 320 to being passed through and eliminated by the second sensing section 131 before reaching the first sensing section 121, so as to prevent the interferant 320 from affecting the sensing result of the physiological parameter.

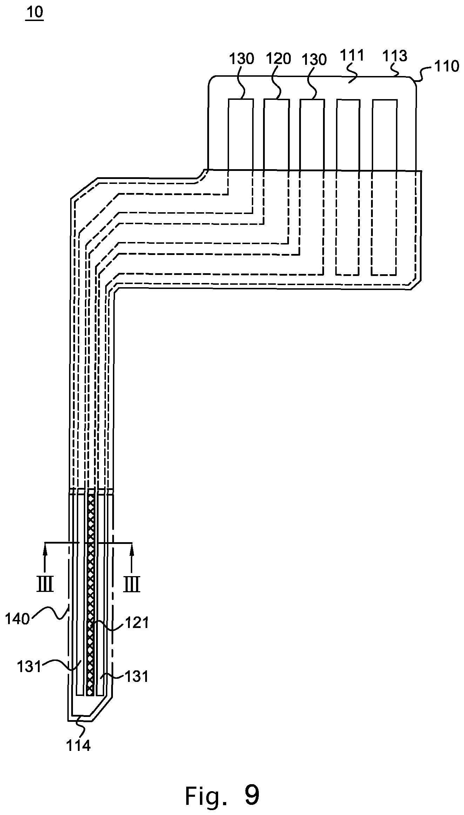

Please refer to Fig. 9, which is a front schematic diagram of the third embodiment of the micro biosensor 10 of the present invention. Because a rear view of the third embodiment is identical to that of the first embodiment and the second embodiment, the back schematic diagram of the third embodiment is omitted. In the third embodiment, the micro biosensor 10 has two second working electrodes 130. The first working electrode 120 and the two second working electrodes 130 extend from the first end 113 to the second end 114 of the substrate 110, and the two second working electrodes 130 respectively extend along the two opposite sides of the first working electrode 120. A cut view along the section line III-IIIin Fig. 9 is the same as Fig.2, where the two second sensing sections 131 are configured adjacent to the first sensing section 121.

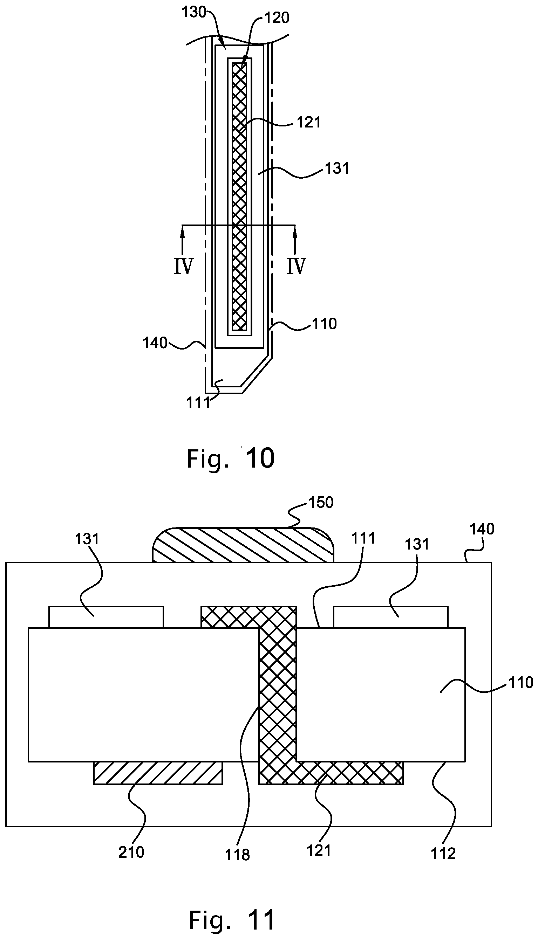

Please refer to Figs. 10 and 11, wherein Fig. 10 is a front schematic diagram of the fourth embodiment of the sensing area of the sensing structure in the micro biosensor of the present invention, and Fig. 11 is a sectional schematic diagram of a cut view of the micro biosensor along the section line IV-IV in Fig. 10. Fig. 10 only shows the sensing area of the micro biosensor 10, and does not show the signal output area and the insulating area. In the fourth embodiment, the second sensing section 131 of the second working electrode 130 can be configured adjacent to the four sides of the first sensing section 121 of the first working electrode 120. Specifically, the second sensing section 131 can extend along the first sensing section 121 to completely surround the first sensing section 121, and has a gap with the first sensing section 121. It can be seen from Fig. 11, there is a through hole 118 on the substrate 110 of the fourth embodiment, where the first sensing section 121 can be extended from the first surface 111 to the second surface 112 of the substrate 110 through the through hole 118 of substrate 110, and the isolated layer 150 is disposed with respect to at least the top of the first sensing section 121. The first working electrode 120 extended from the second surface 112 can return to the first surface 111 through another through hole on the substrate in the signal output area.

It can be seen that the length of the second sensing section 131 may be altered corresponding to the first sensing section 121. Therefore, in order to effectively reduce the influence of the interferant on the measurement, the aforementioned phrase "the second sensing section 131 is adjacent to at least one side of the first sensing section 121" specifically refers that a ratio of the portion of the periphery of the first sensing section 121 adjacent to the second sensing section 131 to a total of the periphery of the first sensing section ranges from 30% to 100%.

Furthermore, as mentioned above, in order for the interference eliminating range 2S of the second sensing section 131 to contact the surroundings of the first sensing section 121 and at least partially overlap the measurement range 1S of the first sensing section 121, a gap between the second sensing section 131 and the first sensing section 121 in the sensing area 116 of the micro biosensor 10 of the present invention in all preceding embodiments is no larger than 0.5 mm. Preferably, the gap between the second sensing section 131 and the first sensing section 121 is no larger than 0.2 mm; more preferably, the gap ranges from 0.01 mm to 0.2 mm; still more preferably, the gap ranges from 0.01 mm to 0.1 mm; and further preferably, the gap ranges from 0.02 mm to 0.05 mm.

In the preceding embodiments, the micro biosensor 10 of the present invention further includes a counter electrode 210 configured on the second surface 112 of the substrate 110, and extended from the first end 113 to the second end 114. The counter electrode 210 is coupled to at least one of the first working electrode 120 and the second working electrode 130, for measuring the physiological signal by cooperating with the first working electrode 120, and consuming the interferant by cooperating with the second working electrode 130. The counter electrode 210 can also function as a reference electrode based on the material it used. Specifically, the counter electrode 210 of the present invention can form an electronic circuit with the first working electrode 120 to freely flow the current on the first working electrode 120, to ensure that the counter electrode 210 can also provide a stable relative potential as a reference potential, while the electrochemical reaction occurs on the first working electrode 120. In another embodiment, the micro biosensor of the present invention can include two counter electrodes, and/or the counter electrode also can be configured on the first surface 111 of the substrate 110 (figure not shown). In a further embodiment, in addition to the counter electrode, the micro biosensor of the present invention also includes a reference electrode used for providing a reference potential. Specifically, the counter electrode and the reference electrode are separate and not electrically connected, and the counter electrode is coupled to the first working electrode 120 and/or the second working electrode 130. The counter electrode and the reference electrode can be both configured on the first surface 111 or the second surface 112 of the substrate 110 (figure not shown), or respectively configured on different surfaces of the substrate 110.

In the preceding embodiments, the first conductive material 1C of the first sensing section 121 can be the same as the second conductive material 2C of the second sensing section 131. In this embodiment, the chemical reagent is configured only to cover the first sensing section 121 of the first working electrode 120, or an interferant elimination layer can be additionally added on the second sensing section 131 of the second working electrode 130, followed by the chemical reagent is coated on the first sensing section 121 of the first working electrode 120 and the interferant elimination layer based on the convenience of the process, etc., which still meets the purpose of consuming the interferant through the second sensing section 131 to obtain the accurate physiological signals.

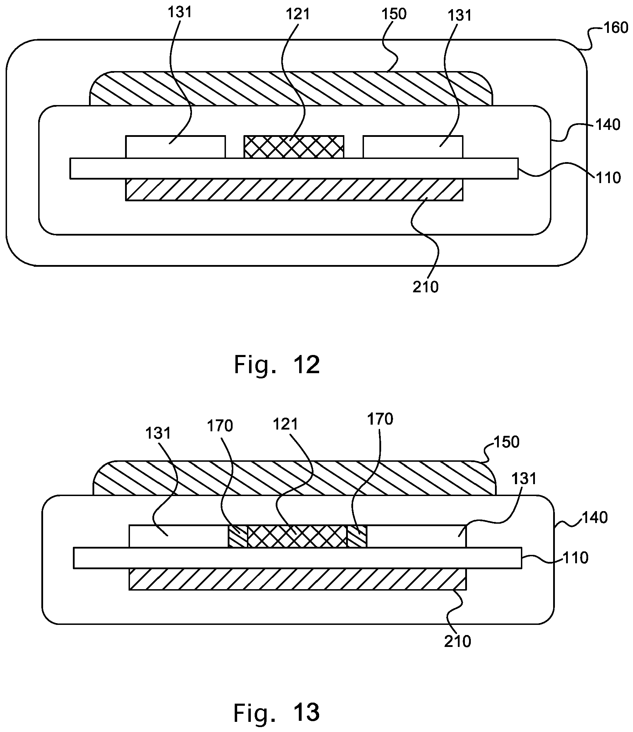

In the preceding embodiments, the micro biosensor 10 of the present invention further includes a second functional membrane 160 as shown in Fig. 12. The second functional membrane 160 is covered the first functional membrane 140 and the isolated layer 150, to wrap the substrate 110, the first sensing section 121, the second sensing section 131, the counter electrode 210, the first functional membrane 140 and the isolated layer 150. The second functional membrane 160 can be used as a protective layer of the micro biosensor 10. Because the sensing structure of the micro biosensor 10 is a flat plate structure and the structure is relatively thin, the stress on its left and right sidedges is stronger. The thickness of the second functional membrane 160 needs to be thick enough on the sidedges of the sensing structure, for example, between 20 µm and 30 µm, to protect the sensing structure from cracking to prevent the condition of leaking current. Therefore, the second functional membrane 160 can further increase the structural strength of the micro biosensor 10. Furthermore, in addition to the protective layer, the second functional membrane 160 can be used as a storage layer for a small amount of tissue fluid. When the user performs strenuous exercise, there is a significant relative displacement between the micro biosensor and the subcutaneous tissue, and the second functional membrane can provide a buffer function to avoid signal clutter.

In the preceding embodiments, the micro biosensor 10 of the present invention further includes a filler 170 as shown in Fig. 13. The filler 170 is filled between the first working electrode 120 and the second working electrode 130, and has a top surface. The top surface of the filler 170, the top surface of the first sensing section 121 of the first working electrode 120 and the top surface of the second sensing section 131 of the second working electrode 130 are coplanar, which causes the electrode surfaces of the first working electrode 120 and the second working electrode 130 to be flat, so that the subsequent coating membrane on the electrode surfaces is flat. In addition, the material of the filler 170 is not limited to be the same material as the isolated layer 150, and the filler 170 and the isolated layer 150 are separated by the first functional membrane 140. However, in another embodiment, if the material of the filler 170 is the same as that of the isolated layer 150, the filler 170 can continue to extend upward to form the isolated layer 150, so that the filler 170 and the isolated layer 150 are integrally formed (figure not shown).

In the preceding embodiments, the sensing structure of the micro biosensor 10 of the present invention further can include the first working electrode 120, the second working electrode 130, the counter electrode 210, the first functional membrane 140, the isolated layer 150, the second functional membrane 160 and the filler 170 formed in the sensing area 116 of the substrate 110. In another embodiment, the sensing structure of the micro biosensor 10 of the present invention further can include the first working electrode 120, the second working electrode 130, the counter electrode 210, the reference electrode, the first functional membrane 140, the isolated layer 150, the second functional membrane 160 and the filler 170 formed in the sensing area 116 of the substrate 110.

To sum up, the isolated layer in the micro biosensor of the present invention can isolate at least part of the interferant to delineate or program the diffusive path of the interferant as one passing through the second working electrode first, to gain an increased opportunity of the interferant to interact with the second sensing section of the second working electrode to consume the interferant, so that the interference on the measurement of the first sensing section caused by the interferant is reduced, and thus the micro biosensor can measure more accurate physiological parameters.

The present invention is an innovative invention and has great industrial value, so that the application is filed according to the law. In addition, the present invention can be modified in any way by those skilled in the art without departing from the scope of protection as claimed in the appended claims.

### REFERENCE NUMERALS

10: micro biosensor
110: substrate
111: first surface
112: second surface
113: first end
114: second end
115: signal output area
116: sensing area
117: insulating area
118: through hole
120: first working electrode
121: first sensing section
130: second working electrode
131: second sensing section
140: first functional membrane
150: isolated layer
160: second functional membrane
170: filler
210: counter electrode
310: target analyte
320: interferant
1C: first conductive material
2C: second conductive material
1S: measurement range
2S: interference eliminating range
I1: first insulating layer
12: second insulating layer
H: thickness

## Claims

1. A micro biosensor for implantation under a skin to measure a physiological parameter of a target analyte of a biofluid and reduce an interference of an interferant of the biofluid on the measurement by an electrochemical reaction, comprising:
a substrate being a sheet and having a first surface and a second surface which are oppositely configured;
a first working electrode at least including a first sensing section configured on the first surface of the substrate, wherein the first sensing section of the first working electrode at least includes a first conductive material;
at least one second working electrode configured on the first surface of the substrate and including a second sensing section, and the second sensing section is configured adjacent to at least one side of the first sensing section, wherein the second sensing section of the second working electrode includes a second conductive material different from the first conductive material;
a first functional membrane covering the first sensing section of the first working electrode and the second sensing section of the second working electrode, and the first functional membrane includes a chemical reagent at least covering a part of the first conductive material to define an active surface of the first sensing section, for reacting with the target analyte of the biofluid so as to obtain a resultant; and
an isolated layer at least configured with respect to at least a part of the active surface of the first sensing section of the first working electrode to optimize a diffusive path of the interferant of the biofluid as one passing through the second sensing section of the second working electrode,
wherein the biofluid diffuses to the second sensing section over a time period, and then diffuses to the first sensing section after passing through the second sensing section;
wherein when the first working electrode is driven by a first working voltage, the first sensing section reacts with the resultant for outputting a physiological signal corresponding to the physiological parameter of the target analyte; and
when the second working electrode is driven by a second working voltage, the second sensing section consumes the interferant of the biofluid by the electrochemical reaction during the time period, and a remaining part of the biofluid diffuses to the first sensing section of the first working electrode after passing through the second sensing section, for reducing the interference of the interferant to the physiological signal.

2. The micro biosensor as claimed in Claim 1, wherein the isolated layer is configured on the first functional membrane and at least with respect to the first sensing section of the first working electrode to at least shield a part of the active surface and to isolate the interferant from diffusing to the active surface directly.

3. The micro biosensor as claimed in Claim 1, wherein the isolated layer is 0.5-10 times an area of the active surface of the first sensing section.

4. The micro biosensor as claimed in Claim 1, wherein the isolated layer is further configured with respect to the second sensing section of the second working electrode to at least shield a part of the second sensing section.

5. The micro biosensor as claimed in Claim 1, wherein the isolated layer has a thickness ranging from 1-80 µm.

6. The micro biosensor as claimed in Claim 1, wherein the time period ranges from 10 seconds to 15 minutes.

7. The micro biosensor as claimed in Claim 1, wherein the first working electrode is driven by the first working voltage to allow the first sensing section to have a measurement range, and the second working electrode is driven by the second working voltage to allow the second sensing section to have an interference eliminating range contacting a surrounding of the first working electrode and at least partially overlapping with the measurement range.

8. The micro biosensor as claimed in Claim 1, wherein the second sensing section of the second working electrode is configured adjacent to at least two sides of the first sensing section.

9. The micro biosensor as claimed in Claim 8, wherein the isolated layer is further configured with respect to the second sensing section to at least shield a part of the second sensing section configured adjacent to the at least two sides of the first sensing section.

10. The micro biosensor as claimed in Claim 1, wherein when the first working electrode is driven by the first working voltage, the first conductive material has a first sensitivity to the resultant, and when the second working electrode is driven by the second working voltage, the second conductive material has a second sensitivity, which is smaller than the first sensitivity, to the resultant.

11. The micro biosensor as claimed in Claim 1, wherein the first conductive material is platinum, and the second conductive material is carbon.

12. The micro biosensor as claimed in Claim 1, wherein the isolated layer is configured on the first functional membrane, the first functional membrane has a thickness defined by a distance between the isolated layer and the active surface of the first sensing section, and the thickness is no less than 0.05 µm and no larger than 50 µm.

13. A micro biosensor for implantation under a skin to measure a physiological parameter of a target analyte of a biofluid and reduce an interference of an interferant of the biofluid on the measurement by an electrochemical reaction, comprising:
a substrate having a first surface and a second surface which are oppositely configured;
a first working electrode at least including a first sensing section configured on the first surface of the substrate, for measuring the physiological parameter of the target analyte;
at least one second working electrode configured on the first surface of the substrate and including a second sensing section, and the second sensing section is configured adjacent to at least one side of the first sensing section for consuming the interferant by the electrochemical reaction;
a first functional membrane covering the first sensing section of the first working electrode and the second sensing section of the second working electrode, and the first functional membrane includes a chemical reagent at least covering a part of the first sensing section to define an active surface for reacting with the target analyte of the biofluid so as to obtain a resultant; and
an isolated layer at least configured with respect to at least a part of the active surface to delineate a diffusive path of the interferant as one passing through the second sensing section of the second working electrode;
wherein when the first working electrode is driven by a first working voltage, the first sensing section reacts with the resultant for outputting a physiological signal corresponding to the physiological parameter of the target analyte; and
when the second working electrode is driven by a second working voltage, the second sensing section consumes the interferant of the biofluid by the electrochemical reaction, and a remaining part of the biofluid diffuses to the first sensing section after passing through the second sensing section, for reducing the interference of the interferant to the physiological signal.

14. The micro biosensor as claimed in Claim 13, wherein the isolated layer is configured on the first functional membrane and at least with respect to the first sensing section of the first working electrode to at least shield a part of the active surface and to isolate the interferant from diffusing to the active surface directly.

15. The micro biosensor as claimed in Claim 13, wherein the isolated layer is further configured with respect to the second sensing section of the second working electrode to at least shield a part of the second sensing section.

16. The micro biosensor as claimed in Claim 13, wherein the second sensing section is configured adjacent to the at least one side of the first sensing section with a gap, and the gap is no larger than 0.5 mm.

17. The micro biosensor as claimed in Claim 13, wherein a number of the second working electrode is two, and the two second sensing sections of the two second working electrodes are respectively configured adjacent to two opposite sides of the first sensing section.

18. The micro biosensor as claimed in Claim 13, wherein a side of the second sensing section extends along a periphery of the first sensing section, and a part of the periphery of the first sensing section adjacent to the second sensing section accounts for 30%-100% of a total length of the periphery of the first sensing section.

19. The micro biosensor as claimed in Claim 13, further comprising at least one counter electrode, wherein the counter electrode configures on the second surface, and couples to at least one of the first working electrode and the second working electrode.

20. The micro biosensor as claimed in Claim 13, wherein the chemical reagent is further covering a part of the second sensing section of the second working electrode.

21. The micro biosensor as claimed in Claim 13, further comprising:
a second functional membrane wrapping the first surface and the second surface of the substrate to cover the first functional membrane and the isolated layer.

22. The micro biosensor as claimed in Claim 13, wherein the first functional membrane further includes a filler, and the filler is filled between the first working electrode and the second working electrode.

23. A sensing structure of a micro biosensor for implantation under a skin to measure a physiological parameter of a target analyte of a biofluid and reduce an interference of an interferant of the biofluid on the measurement by an electrochemical reaction, comprising:
a substrate having a surface;
a first working electrode configured on the surface of the substrate, and having an active surface;
at least one second working electrode configured on the surface of the substrate and adjacent to at least one side of the first working electrode, for consuming the interferant by the electrochemical reaction; and
an isolated layer at least configured with respect to at least a part of the active surface to program a diffusive distribution of the interferant when the biofluid flows through the second working electrode,
wherein at least the interferant of the biofluid passes through the second working electrode over a time period and is consumed by the second working electrode by the electrochemical reaction.

24. The sensing structure as claimed in Claim 23, further comprising:
a first functional membrane configured between the first working electrode and the isolated layer, and wrapping the substrate, the first working electrode and the second working electrode, wherein the first functional membrane comprises a chemical reagent covering a part of the first working electrode to define the active surface for reacting with the target analyte of the biofluid so as to obtain a resultant.

25. The sensing structure as claimed in Claim 24, wherein:
when the first working electrode is driven by a first working voltage, the first working electrode reacts with the resultant for outputting a physiological signal corresponding to the physiological parameter of the target analyte; and
when the second working electrode is driven by a second working voltage, the second working electrode consumes the interferant by performing the electrochemical reaction within the time period for reducing the interference of the interferant to the physiological signal.

26. The sensing structure as claimed in Claim 25, wherein the first working voltage is 0.2-0.8 volt, and the second working voltage is 0.2-0.8 volt.

27. The sensing structure as claimed in Claim 23, wherein the diffusive distribution is that at least a remaining part of the biofluid reaches the first working electrode after passing through the second working electrode.
